## Europäisches Patentamt

## European Patent Office

## Office européen des brevets

(19)

(11) Veröffentlichungsnummer: **0 184 748**
**B1**

# EUROPÄISCHE PATENTSCHRIFT

(12)

(45) Veröffentlichungstag der Patentschrift:
01.03.89

(21) Anmeldenummer: 85115260.3

(22) Anmeldetag: 02.12.85

(51) Int. Cl.⁴: **A 61 C 19/04**, G 01 N 3/30, G 01 L 25/00

(54) Testvorrichtung für ein zahnärztliches Perkussionsinstrument.

(30) Priorität: 13.12.84 DE 3445507

(43) Veröffentlichungstag der Anmeldung:
18.06.86 Patentblatt 86/25

(45) Bekanntmachung des Hinweises auf die Patenterteilung:
01.03.89 Patentblatt 89/9

(84) Benannte Vertragsstaaten:
CH DE FR IT LI SE

(56) Entgegenhaltungen:
DE-U- 1 956 091
US-A- 3 007 336

REVIEW SCIENTIFIC INSTRUMENTS, Band 50, Nr. 12, Dezember 1979, Seiten 1645-1648, American Institute of Physics, New York, US; J.L. SMITH et al.: "Instrument for measuring tooth mobility"

(73) Patentinhaber: Siemens Aktiengesellschaft Berlin und München, Wittelsbacherplatz 2, D-8000 München 2 (DE)

(72) Erfinder: Wohlgemuth, Jürgen, Brüder-Knaus-Strasse 61, D-6100 Darmstadt (DE)

## Beschreibung

Die Erfindung betrifft eine Testvorrichtung für ein zahnärztliches Perkussionsinstrument, welches einen beweglich gelagerten Stössel enthält, der mit Hilfe eines vorzugsweise magnetischen Antriebes und einer den Antrieb steuernden Elektronik auf eine definierte Geschwindigkeit beschleunigt und danach im freien Flug mit gleichbleibender Geschwindigkeit auf ein Messobjekt zu bewegt und nach Aufprall des Stösselendes auf dem Objekt mittels des Antriebes wieder in seine Ausgangsposition zurückgestellt wird.

Ein derartiges Perkussionsinstrument, mit dem vorzugsweise der Lockerungsgrad von Zähnen im menschlichen Zahnhalteapparat bestimmt werden kann, ist beispielsweise in der DE-32 15 530 beschrieben.

Bei Benutzung solcher Instrumente kann es vorkommen, dass aufgrund eines Defekts am Instrument oder in der Elektronik Fehlmessungen durchgeführt werden, ohne dass diese vom Benutzer als solche erkannt werden.

Mit der im Anspruch 1 angegebenen Testvorrichtung kann die Funktion des kompletten Perkussionsgerätes vor der eigentlichen Messung am Patienten getestet und eventuelle Defekte am Instrument oder an der Elektronik leicht festgestellt werden.

Vorteilhafte Ausgestaltungen und Weiterbildungen sind in den Unteransprüchen enthalten. Mehrere Ausführungsformen der erfindungsgemässen Testvorrichtung werden nachfolgend anhand der Zeichnung näher erläutert. Es zeigen:

Figur 1 eine einfache Ausführungsform der erfindungsgemässen Testvorrichtung,

Figuren 2 und 3 zwei verschiedene Varianten einer Testvorrichtung,

Figur 4 einen Teil der in Figur 3 gezeigten Testvorrichtung,

Figur 5 eine weitere Variante einer Testvorrichtung.

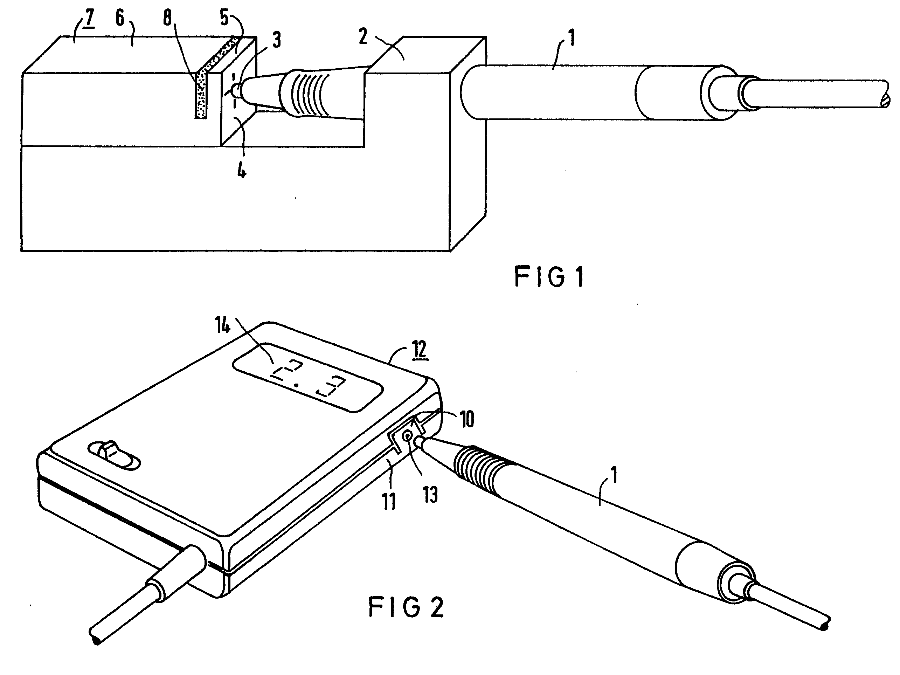

Die Figur 1 zeigt die erfindungsgemässe Testvorrichtung in einer einfachen Ausführung. Die Testvorrichtung ist für ein in der Figur mit 1 bezeichnetes Perkussionsinstrument bestimmt, dessen Aufbau in der deutschen Patentanmeldung P32 15 530 beschrieben ist. Das Instrument ist waagerecht in einer mit 2 bezeichneten Halterung so eingespannt, dass für einen Testvorgang das Stösselende 3 an einer senkrechten Fläche 4 eines Testkörpers 5 zur Anlage kommen kann. Der Testkörper 5 weist im Verhältnis zu einem mit ihm verbundenen massiven Träger 6 eine gewisse Elastizität auf, die der des zu prüfenden Messobjektes, im vorliegenden Falle also der Elastizität eines in einem gesunden Zahnbett verankerten Zahnes, entspricht. Der Testkörper 5 hat also definierte gleichbleibende elasto-mechanische Eigenschaften, die denen eines gesunden Zahnes in seinem Zahnbett entsprechen. Diese Elastizität wird im gezeigten Ausführungsbeispiel dadurch erreicht, dass ein allgemein mit 7 bezeichneter quaderförmiger Grundkörper aus Hartgummi, Hartgewebe oder einem anderen dafür geeigneten Werkstoff im vorderen, dem Instrument 1 zugewandten Bereich mit einem Querschlitz 8 versehen ist, der den Körper in einen festen (starren) Körperabschnitt 6 und einen demgegenüber federelastischen Körperabschnitt 5 unterteilt. Um eine gewisse Dämpfung des Körperabschnittes 5 zu erzielen, ist der Schlitz 8 vorteilhafterweise mit einem geeigneten Dämpfungsmaterial aus Weichgummi, Schaumstoff oder dergleichen ausgefüllt. Im gezeigten Ausführungsbeispiel bilden Testkörper 5 und Träger 6 zusammen mit der Instrumentenhalterung 2 eine Baueinheit. Dies ist zwar vorteilhaft, jedoch nicht, wie z.B Figur 2 zeigt, zwingend notwendig.

Der elasto-mechanisch angeordnete Testkörper kann verschiedenartig ausgebildet und angeordnet sein. Beim Ausführungsbeispiel nach Figur 2 ist der Testkörper eine federnde Zunge 10, die durch teilweises Ausstanzen einer definierten Fläche einer waagerechten Wandung 11 eines Gehäuses 12 gebildet ist, die gegenüber den übrigen Gehäuseflächen die oben erwähnten elastischen Eigenschaften aufweist. Zweckmässig ist es, die federnde Zunge 10 mit einer Markierung 13 zu versehen, an die die Stösselspitze des Instruments 1 für den Testvorgang angelegt werden kann.

Besonders vorteilhaft ist es, den Testkörper, im Ausführungsbeispiel nach Figur 2 also die federnde Zunge 10, in das Gehäuse 12 zu integrieren, in welchem die Steuer- und Auswerteelektronik für das Instrument sowie ein die Testwerte anzeigendes Display 14 vorgesehen ist. Die Testmessungen können so am Display abgelesen und mit bestimmten, die Gerätefunktion festlegenden Werten überprüft werden.

Anstelle der teilweisen Ausstanzung eines bestimmten Gehäusebereichs kann auch an einer bestimmten Stelle des Gehäuses eine Fläche mit gegenüber den übrigen Gehäuseteilen gleichbleibender Materialschwächung oder Materialverdikkung vorgesehen sein. Diese Fläche muss dann ebenfalls die oben erwähnten Eigenschaften aufweisen.

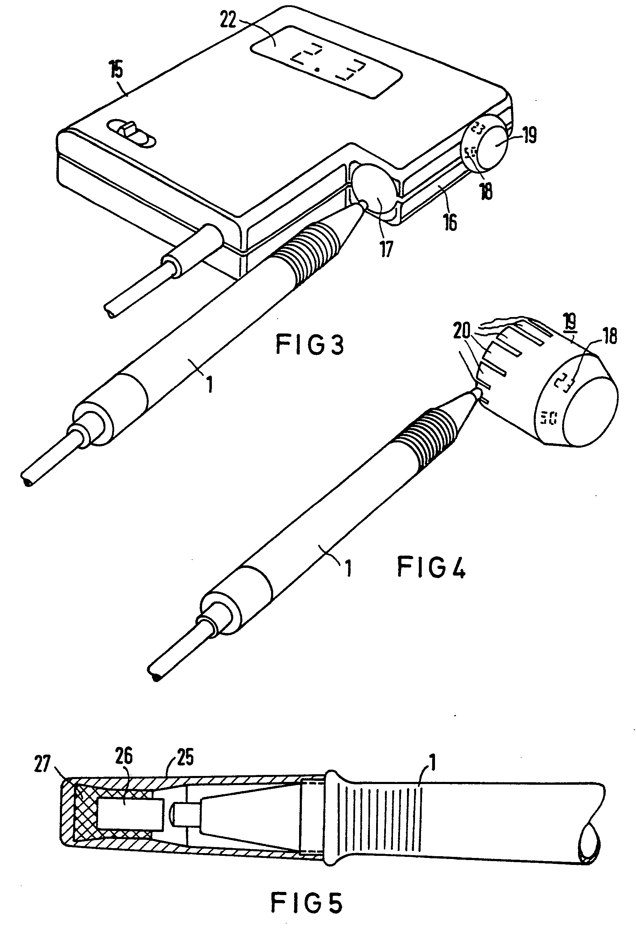

Die Figur 3 zeigt eine weitere Ausführungsform einer Testvorrichtung, die, ähnlich wie in Figur 1, eine Instrumentenhalterung aufweist. Darüber hinaus weist diese Testvorrichtung eine Vielzahl von auf unterschiedliche Werte einstellbare Testkörper auf. Die Instrumentenhalterung ist hier in das Gehäuse 15 integriert und als hülsenförmiges, längsgeschlitztes Teil 16 ausgebildet, indem das Instrument 1 für eine waagerechte Halterung eingeführt werden kann. Quer zur Aufnahmeöffnung 17 für das Instrument ist ein mit einer Skala 18 versehenes Teil 19, welches in Figur 4 vollständig dargestellt ist, drehbar gelagert. Das Teil 19 besteht aus einer dünnen Hülse, die am einen Ende offen ist und am Umfang mehrere, unterschiedlich lange federnde Zungen 20 und am anderen, stirnseitig geschlossenen Ende die Skala mit den Zungen 20 zugeordnete, bestimmten Testwerten entsprechende Markierungen enthält. Durch Drehen der Hülse 19 können so individuelle Testwerte eingestellt werden, die dann, wenn das Instrument und die Elektronik einwandfrei funktionieren, am Display 22 angezeigt werden müssen, wie dies bespielsweise in Figur 3 dargestellt ist.

Anstelle der Hülse kann auch ein längs verschiebbares Trägerteil mit mehreren, unterschiedlich stark federnden Zungen vorgesehen sein.

Die Figur 5 zeigt eine weitere vorteilhafte Variante einer Testvorrichtung. Bei dieser Ausführungsform ist die Testvorrichtung Bestandteil einer auf das Instrument 1 aufsteckbaren und im aufgesteckten Zustand das Stösselende schützend umgebenden Kappe 25. Im Inneren der hülsenförmigen Kappe ist ein Körper 26 mit einer dem Prüfobjekt, im vorliegenden Anwendungsfall also einem Zahn, entsprechenden Masse in einer elastischen Halterung 27 eingebettet. Die Haltung 27 weist, wie bei den zuvor aufgezeigten Ausführungsbeispielen bereits erläutert, dem Prüfobjekt entsprechende Dämpfungs- und Federeigenschaften auf.

## Patentansprüche

1. Testvorrichtung zur Funktionsüberprüfung von zahnärztlichen Perkussionsinstrumenten (1), welche einen beweglich gelagerten Stössel enthalten, der mit Hilfe eines Antriebs und einer den Antrieb steuernden Elektronik auf eine definierte Geschwindigkeit beschleunigt, danach im freien Flug mit gleichbleibender Geschwindigkeit auf ein zu perkutierendes Objekt (Zahn) zubewegt und nach Aufprall des Stösselendes (3) auf dem Objekt wieder in seine Ausgangsposition zurückgestellt wird, dadurch gekennzeichnet, dass baulich getrennt vom Perkussionsinstrument (1) ein an einem Träger (6, 11, 12, 19, 25) angeordneter Testkörper (5, 10, 20, 26) vorgesehen ist, der hinsichtlich seiner Ausbildung und/oder Anordnung am Träger elasto-mechanische Eigenschaften besitzt, die dem zu perkutierenden Objekt entsprechen und dass der Testkörper mit einer Anlagefläche (4) versehen ist, an der das Stösselende (3) des Perkussionsinstruments (1) bei einer Funktionsüberprüfung zur Anlage gelangt.

2. Testvorrichtung nach Anspruch 1, dadurch gekennzeichnet, dass der Testkörper (5) Bestandteil des Trägers (6) ist und durch einen mit Dämpfungsmaterial ausgefüllten Schlitz (8) in einem Körper (7) aus Vollmaterial gebildet ist.

3. Testvorrichtung nach Anspruch 1, dadurch gekennzeichnet, dass der Testkörper (10) Bestandteil des Trägers (12) ist und durch eine in einem definierten Flächenabschnitt in einer Gehäusewandung (11) eines den Träger bildenden Gehäuses (12) vorgesehene Materialschwächung oder -verdickung gebildet ist.

4. Testvorrichtung nach Anspruch 1, dadurch gekennzeichnet, dass der Testkörper Bestandteil des Trägers (12) ist und durch eine federnde Zunge (10) gebildet ist.

5. Testvorrichtung nach Anspruch 4, dadurch gekennzeichnet, dass die federnde Zunge (10) durch die teilweise Ausstanzung in einem den Träger bildenden Gehäuse (12) gebildet ist.

6. Testvorrichtung nach Anspruch 3 oder 4, dadurch gekennzeichnet, dass das Gehäuse (12) die Auswerteelektronik für das Instrument und ein Anzeigedisplay (14) aufnimmt.

7. Testvorrichtung nach einem der Ansprüche 1 bis 6, dadurch gekennzeichnet, dass der Testkörper (5, 20) an einer Halterung (2, 16) für das Instrument (1) angeordnet ist, welches so ausgebildet bzw. angeordnet ist, dass das Instrument waagerecht und das Stösselende (3) des Instruments (1) auf eine senkrechte Anlagefläche (4) des Testkörpers (5, 20) ausgerichtet ist.

8. Testvorrichtung nach einem der Ansprüche 1 bis 7, dadurch gekennzeichnet, dass mehrere Testkörper (20) mit unterschiedlichen elasto-mechanischen Eigenschaften an einem gemeinsamen Träger (19) angeordnet sind.

9. Testvorrichtung nach Anspruch 8, dadurch gekennzeichnet, dass die vom Träger (19) aufgenommenen Testkörper (20) derart verstellbar angeordnet sind, dass sie sich jeweils auf das Stösselende (3) des von der Halterung (16) aufgenommenen Instruments (1) ausrichten lassen.

10. Testvorrichtung nach Anspruch 9, dadurch gekennzeichnet, dass als Träger eine drehbare Hülse (19) vorgesehen ist, die am einen Ende am Umfang mehrere, durch unterschiedlich lange Längsschlitze gebildete Zungen (20) und am anderen Ende den Zungen (20) zugeordnete, definierten Messwerten entsprechende Markierungen (18) aufweist.

11. Testvorrichtung nach Anspruch 10, dadurch gekennzeichnet, dass die Hülse (19) quer zu einer hülsenförmigen Halterung (16) für das Instrument angeordnet ist.

12. Testvorrichtung nach Anspruch 1, dadurch gekennzeichnet, dass sie Bestandteil einer auf das Instrument (1) aufsetzbaren Kappe (25) ist.

13. Testvorrichtung nach Anspruch 12, dadurch gekennzeichnet, dass die Kappe (25) so ausgebildet ist, dass sie auf das das Stösselende aufweisende Ende des Instruments (1) aufsteckbar ist, wobei im aufgesteckten Zustand der im Inneren der Kappe mittels eines geeigneten Trägermaterials (27) angeordnete Testkörper (26) dem Stösselende korrespondierend gegenübersteht.

## Revendications

1. Dispositif d'essai servant à contrôler le fonctionnement d'instruments dentaires à percussion contenant une pointe de percussion, qui est montée de manière à être déplaçable, est accélérée au moyen d'un dispositif d'entraînement et d'un système électronique commandant le dispositif d'entraînement, pour être amenée à une vitesse définie, et est ensuite déplacée en vol libre à une vitesse constante en direction d'un objet devant être percuté (dent) et est à nouveau rétractée dans sa position initiale après l'impact de son extrémité (3) sur l'objet, caractérisé par le fait qu'il est prévu, dans une disposition séparée de l'instrument à percussion (1) du point de vue construction, un corps d'essai (5, 6, 20, 26) qui est disposé sur un support (6, 12, 19, 25) et dont la réalisation et/ou la disposition sur le support présentent des propriétés élasto-mécaniques, qui correspondent à l'objet à percuter, et que le corps d'essai comporte une surface d'appui (4), contre laquelle l'extrémité (3) de la pointe de percussion de l'instrument à percussion (1) vient s'appliquer lors du contrôle du fonctionnement.

2. Dispositif d'essai suivant la revendication 1, caractérisé par le fait que le corps d'essai (5) fait partie du support (6) et est formé par une fente (8) remplie par un matériau d'amortissement et formée dans un corps massif (7) réalisé en un matériau plein.

3. Dispositif d'essai suivant la revendication 1, caractérisé par le fait que le corps d'essai (10) fait partie du support (12) et est formé par un amincissement ou un épaississement du matériau prévu dans une section de surface définie dans une paroi (11) d'un boîtier (12) constituant le support.

4. Dispositif d'essai suivant la revendication 1, caractérisé en ce que le corps d'essai fait partie du support (12) et est formé par une languette élastique (10).

5. Dispositif d'essai suivant la revendication 4, caractérisé par le fait que la languette élastique (10) est formée au moyen d'un découpage partiel dans un boîtier (12) constituant le support.

6. Dispositif d'essai suivant la revendication 3 ou 4, caractérisé par le fait que le boîtier (12) loge le système électronique d'évaluation pour l'appareil et un dispositif d'affichage (14).

7. Dispositif d'essai suivant l'une des revendications 1 à 6, caractérisé par le fait que le corps d'essai (5, 20) est monté dans un dispositif (2, 16) de retenue de l'instrument (1), qui est agencé ou disposé de telle sorte que l'instrument est horizontal et que l'extrémité (3) de la pointe de percussion de l'instrument (1) est dirigée vers une surface verticale d'application (4) du corps d'essai (5, 20).

8. Dispositif d'essai suivant l'une des revendications 1 à 7, caractérisé par le fait que plusieurs corps d'essai (20) possédant des propiétés élasto-mécaniques différentes sont disposés sur un support commun (19).

9. Dispositif d'essai suivant la revendication 8, caractérisé par le fait que les corps d'essai (20) portés par le support (19) sont disposés de manière à être déplaçables de telle sorte qu'on peut les aligner respectivement sur l'extrémité (3) de la pointe de percussion de l'instrument (1) logé dans le dispositif de support (16).

10. Dispositif d'essai selon la revendication 9, caractérisé en ce qu'il est prévu, comme support, une douille rotative (19), dont une extrémité comporte, sur son pourtour, plusieurs languettes (20) formées par des fentes longitudinales possédant des longueurs différentes, et dont l'autre extrémité comporte des marques (18) associées aux languettes (20) et correspondant à des valeurs de mesure définies.

11. Dispositif d'essai suivant la revendication 10, caractérisé par le fait que la douille (19) est disposée transversalement par rapport à un dispositif en forme de manchon (16) servant à supporter l'instrument.

12. Dispositif d'essai suivant la revendication 1, caractérisé par le fait qu'il fait partie d'un capuchon (25) pouvant être placé sur l'instrument (1).

13. Dispositif d'essai suivant la revendication 12, caractérisé par le fait que le capuchon (25) est agencé de telle sorte qu'il peut être enfiché sur l'extrémité de l'instrument (1) portant l'extrémité de la pointe de percussion, auquel cas, à l'état enfiché, le corps d'essai (26) disposé à l'intérieur du capuchon moyennant l'interposition d'un matériau de support approprié (27), est disposé dans une position correspondante en vis-à-vis de l'extrémité de la pointe de percussion.

**Claims**

1. A device for monitoring the functioning of dental percussion instruments (1) that comprise a moveably mounted striker that is accelerated to a predetermined velocity by means of a drive and electronic drive controls and then moves in free flight at constant velocity towards an object (a tooth) to be struck, and after impact of the striker tip (3) on the object is returned to its starting position, characterised in that a testing body (5, 10, 20, 26) is arranged on a carrier (6, 11, 12, 19, 25) structurally separate from the percussion instrument (1) and has, in respect of its structure and/or its arrangement on the carrier, elasto-mechanical properties corresponding to those of the object to be struck, and in that the testing body is provided with a contact surface (4) with which the striker tip (3) of the percussion instrument (1) comes into contact in a functional test.

2. A testing device according to claim 1, characterised in that the testing body (5) forms part of the carrier (6) and comprises a slot (8) filled with attenuating material in a massive body (7) of solid material.

3. A testing device according to claim 1, characterised in that the testing body (10) forms part of the carrier (12) and comprises a weakening or thickening of the material of a particular section of a wall (11) of a housing (12) that forms the carrier.

4. A testing device according to claim 1, characterised in that the testing body forms part of the carrier (12) and comprises a resilient tongue (10).

5. A testing device according to claim 4, characterised in that the resilient tongue (10) is formed by partially stamping out from the housing (12) forming the carrier.

6. A testing device according to claim 3 or claim 4, characterised in that the housing (12) accommodates the evaluation electronics of the instrument and an indicator display.

7. A testing device according to any one of claims 1 to 6, characterised in that the testing body (5, 20) is arranged on a holder (2, 16) for the instrument (1) that is so constructed or arranged that the instrument is horizontal and the striker tip (3) of the instrument (1) is directed towards a vertical contact face (4) of the testing body (5, 20).

8. A testing device according to any one of claims 1 to 7, characterised in that a plurality of testing bodies (20) with different elasto-mechanical properties are arranged on a common carrier (19).

9. A testing device according to claim 8, characterised in that the testing bodies (20) on the carrier (19) are adjustable so that they can each be directed towards the striker tip (3) of the instrument (1) in the holder (16).

10. A testing device according to claim 9, characterised in that the carrier is a rotatable sleeve (19) having at one end a plurality of tongues (20) at its circumference formed by longitudinal slots of different lengths and at its other end markings (18) corresponding to particular measuring values associated with the tongues (20).

11. A testing device according to claim 10, characterised in that the sleeve (19) is arranged transverse to a sleeve-shaped holder (16) for the instrument.

12. A testing device according to claim 1, characterised in that it forms part of a cap (25) that can be fitted on to the instrument (1).

13. A testing device according to claim 12, characterised in that the cap (25) is formed so that it can be fitted on to the end of the instrument (1) having the striker tip so that, when it is attached, the testing body (26) arranged inside the cap by means of a suitable carrier material (27) correspondingly faces the striker tip.

FIG 1

FIG 2

2/2

FIG 3

FIG 4

FIG 5